# FASCICULE DE BREVET EUROPEEN

(11) **EP 4 469 681 B1**
(45) Date de publication et mention de la délivrance du brevet: **29.10.2025**
(21) Numéro de dépôt: 23702398.1
(22) Date de dépôt: 24.01.2023
(51) Int. Cl.: F04B 37/14, A61L 2/04, A61L 2/10, A61L 2/16, F04B 39/16, F04B 41/06, F04C 25/02, F04D 19/04, F04B 19/04

(54) **POMPE À VIDE ET INSTALLATION DE TRAITEMENT ET/OU D'EMBALLAGE DE PRODUITS ALIMENTAIRES**
VAKUUMPUMPE UND ANLAGE ZUR VERARBEITUNG UND/ODER VERPACKUNG VON LEBENSMITTELN
VACUUM PUMP AND INSTALLATION FOR PROCESSING AND/OR PACKAGING OF FOOD PRODUCTS

(30) Priorité: 26.01.2022 CH 772022
(43) Date de publication de la demande: 04.12.2024
(73) Titulaire: Ateliers Busch S.A., 2906 Chevenez (CH)
(72) Inventeur: FELTEN, Nicolas, 90140 Bourogne (FR); LIPPELT, Erik, 79594 Inzlingen (DE)
(74) Mandataire: BOVARD AG
(86) Numéro de dépôt international: PCT/EP2023/051595
(87) Numéro de publication internationale: WO 2023/144102

(56) Documents cités:
- WO-A1-2018/082877
- CN-U- 204 957 080
- FR-A1- 2 968 733
- US-A- 4 993 598
- US-A1- 2017 014 539

## Description

### Domaine technique de l'invention

La présente invention se rapporte aux techniques du vide. Plus précisément, elle concerne une pompe à vide, ainsi qu'une installation de traitement et/ou d'emballage de produits alimentaires.

### État de la technique

L'emballage des aliments sous vide est connu. Elle est réalisée dans des installations comprenant une chambre à vide qu'un ou plusieurs conduits de raccordement relient à une ou plusieurs pompes à vide. La ou les pompes à vide ont pour fonction de produire puis de maintenir un vide dans la chambre à vide. En plus de l'emballage d'aliments, la préparation des aliments peut être réalisée dans une telle chambre à vide.

La très faible pression dans la chambre à vide favorise l'évaporation de liquides présents dans les aliments et/ou leur transformation en aérosols volatiles. Des produits provenant des aliments peuvent ainsi être aspirés notamment sous une forme gazeuse ou sous forme d'aérosols dans le ou les conduits de raccordement, vers la ou les pompes à vide. De ce fait, le ou les conduits de raccordements entre la chambre à vide et la ou les pompes à vide peuvent être progressivement souillés par de la matière alimentaire en provenance des aliments dans la chambre à vide. Il existe alors un risque que des micro-organismes pathogènes tels que des bactéries se développent dans le ou les conduits de raccordements entre la chambre à vide et la ou les pompes à vide, ce qui doit être impérativement évité pour des raisons sanitaires.

Pour éviter que des micro-organismes pathogènes tels que des bactéries se développent dans le ou les conduits de raccordements entre la chambre à vide et la ou les pompes à vide, il est connu de régulièrement débrancher et nettoyer ces conduits de raccordement, ce qui requiert une intervention de maintenance et parfois l'arrêt momentané de l'installation.

Par exemple, des installations d'emballage d'aliments munies de dispositifs de stérilisation sont décrites dans la demande WO 2018/082877. Les dispositifs de stérilisation sont agencés sur les conduits de raccordement entre la chambre à vide et la pompe à vide. Il est aussi connu d'utiliser un filtre antibactérien sur un conduit d'un réseau de distribution de vide comme décrit dans la demande FR 2968733.

### Exposé sommaire de l'invention

L'invention a au moins pour but de permettre d'accroître la sureté sanitaire dans les et/ou autour des installations où un vide est produit par une ou plusieurs pompes à vide.

Selon l'invention, ce but est atteint grâce à une pompe à vide qui comporte une aspiration, un refoulement, un volume intérieur prévu pour être parcouru par les gaz entraînés de l'aspiration au refoulement par le pompage par la pompe à vide, ainsi qu'au moins un élément de stérilisation d'au moins une portion du volume intérieur, et le volume intérieur comporte une zone tampon stérilisable par le ou les éléments de stérilisation, cette zone tampon comprenant un passage pour l'ensemble des gaz entraînés par le pompage par la pompe à vide.

Il est possible que l'élément de stérilisation soit unique ou soit l'un de plusieurs éléments de stérilisation que comporte la pompe à vide. Le ou les éléments de stérilisation peuvent être positionnés de manière à stériliser un foyer potentiel de développement et de dissémination de micro-organismes ou une zone tampon entre une région amont pouvant être en amont de l'aspiration de la pompe et une région aval pouvant être l'atmosphère environnant.

Si la pompe à vide selon l'invention est raccordée à une chambre à vide par un conduit de raccordement, le risque de contamination de la chambre à vide par des micro-organismes depuis la pompe à vide est diminué, en particulier dans le cas où un nettoyage programmé du conduit de raccordement n'est effectué qu'après la date initialement prévue, est incorrectement réalisé ou est omis.

La zone tampon peut empêcher une contamination vers l'amont par des micro-organismes présents en aval de cette zone tampon. Également, des virus par exemple provenant d'aliments dans une chambre à vide peuvent être détruits dans la zone tampon et ainsi ne pas être expulsés dans l'atmosphère environnant, par le refoulement de la pompe à vide.

Lorsqu'elle est prévue, toute la zone tampon est stérilisée. La stérilisation de toute la zone tampon peut toutefois ne concerner qu'un ou plusieurs micro-organismes et/ou qu'un ou plusieurs virus. En ce sens, la stérilisation de toute la zone tampon peut n'être que partielle. Elle peut également être totale en supprimant tout micro-organisme et/ou tout virus dans la zone tampon.

Avantageusement, la zone tampon s'étend jusqu'à l'aspiration de la pompe à vide.

Lorsque tel est le cas, la zone tampon peut empêcher qu'une contamination de l'aspiration de la pompe à vide par des micro-organismes se produise puis se propage en aval de la pompe à vide, depuis l'aspiration de la pompe à vide, et atteigne, par exemple, une chambre à vide où des produits alimentaires sont traités et/ou emballés sous vide.

Avantageusement, la pompe à vide comprend au moins une chambre de pompage où s'effectue l'entraînement des gaz par la pompe à vide, au moins une partie du passage de la zone tampon se trouvant en amont de la chambre de pompage.

Avantageusement, la pompe à vide comprend une grille de filtration des gaz pompés, cette grille se trouvant en amont de la chambre de pompage, au moins une partie du passage de la zone tampon se trouvant en amont de la grille.

Avantageusement, une surface amont parmi deux surfaces principales opposées de la grille est stérilisable par le ou les éléments de stérilisation.

Avantageusement, l'élément de stérilisation est une source de lumière ultraviolette.

La ou les sources de lumière ultraviolette produisent de la lumière ultraviolette seule ou de la lumière ultraviolette et une ou plusieurs autres lumières. Avantageusement, la ou les sources de lumière ultraviolette produisent de la lumière ultraviolette dans une gamme de longueur d'onde allant de 250 nm à 280 nm. Lorsque tel est le cas, la lumière ultraviolette possède une efficacité très élevée de suppression de virus tels que le rotavirus et de plusieurs bactéries pathogènes dont l'Escherichia coli et le staphylocoque doré. De manière plus avantageuse, la ou les sources de lumière ultraviolette produisent de la lumière ultraviolette dans une gamme de longueur d'onde allant de 260 nm à 270 nm. Lorsque tel est le cas, la lumière ultraviolette possède une efficacité encore plus élevée de suppression de virus tels que le rotavirus et de plusieurs bactéries pathogènes dont l'Escherichia coli et le staphylocoque doré.

Avantageusement, la source de lumière ultraviolette est l'une de plusieurs sources de lumière ultraviolette que comporte la pompe à vide et qui sont à même d'éclairer ensemble toute une portion de paroi intérieure délimitant et entourant le passage de la zone tampon. De la sorte, toute cette portion de paroi peut être efficacement désinfectée et la zone tampon peut efficacement jouer son rôle de barrière contre une contamination microbienne entre une zone en amont de la zone tampon et une zone en aval de la zone tampon.

Avantageusement, la pompe à vide comprend un conduit rapporté formant l'aspiration de la pompe à vide, ce conduit rapporté étant pourvu de l'élément de stérilisation. De la sorte, n'importe quelle pompe standard peut être transformée en pompe conforme à l'invention.

Avantageusement, l'élément de stérilisation est un élément chauffant. Cet élément chauffant peut effectuer un chauffage par rayonnement, par exemple de la zone tampon. L'élément chauffant peut aussi être une résistance électrique annulaire entourant et délimitant le passage de la zone tampon.

Avantageusement, la pompe à vide est une pompe lubrifiée comprenant une chambre de pompage où s'effectue l'entraînement des gaz par la pompe à vide, ainsi que de l'huile de lubrification présente notamment dans la chambre de pompage, l'huile de lubrification étant ou comprenant l'élément de stérilisation.

Avantageusement, l'élément de stérilisation est ou comprend un produit chimique antiseptique.

L'invention a également pour objet une installation de traitement et/ou d'emballage de produits alimentaires, qui comprenant une chambre à vide et au moins une pompe à vide telle que définie précédemment, l'aspiration de cette pompe à vide étant raccordée à la chambre à vide.

### Brève description des dessins

D'autres avantages et caractéristiques ressortiront plus clairement de la description qui va suivre de plusieurs modes particuliers de réalisation de l'invention donnés à titre d'exemples non limitatifs et représentés aux dessins annexés, parmi lesquels :
- la figure 1 est une vue en perspective, avec arrachement, d'une pompe à vide selon un premier mode de réalisation de l'invention,
- la figure 2 est un schéma d'une installation qui est conforme à l'invention et qui est plus précisément une installation de traitement et/ou d'emballage de produits alimentaires,
- la figure 3 est une vue en perspective et en coupe d'un sous-ensemble amont d'une pompe à vide selon un deuxième mode de réalisation de l'invention,
- la figure 4 est une vue en perspective et en coupe d'un sous-ensemble amont d'une pompe à vide selon un troisième mode de réalisation de l'invention, et
- la figure 5 est une vue en perspective et en coupe d'une pompe à vide selon un quatrième mode de réalisation de l'invention.

### Description d'un mode préférentiel de l'invention

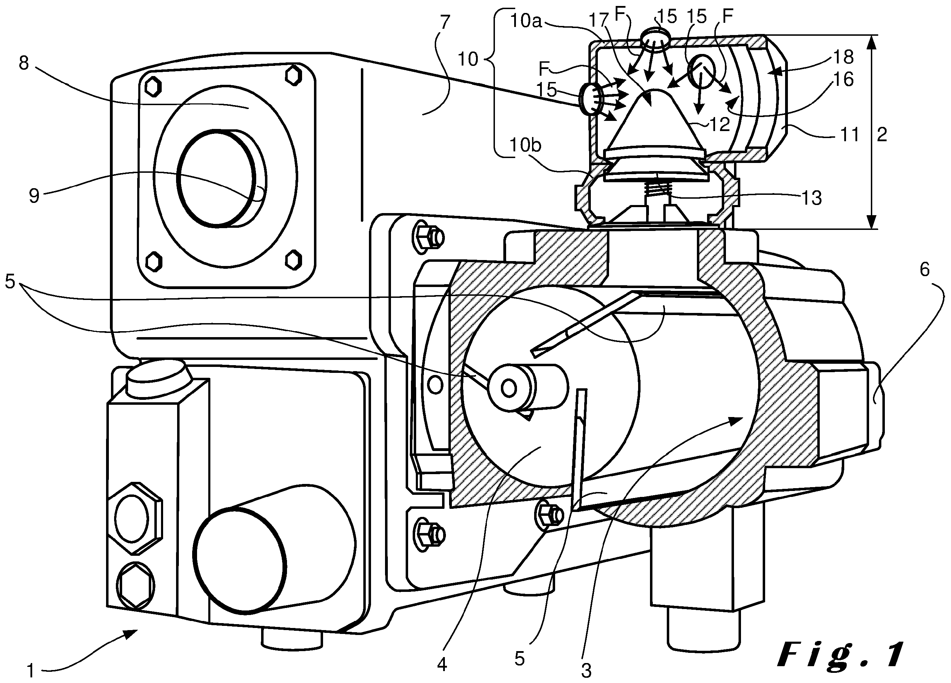

Sur la figure 1, une pompe à vide 1 selon un premier mode de réalisation de l'invention est plus précisément une pompe lubrifiée à palettes. Elle comporte un sous-ensemble amont 2 et une chambre de pompage 3, dans laquelle un rotor 4 pourvu de plusieurs palettes 5 est monté de manière à être rotatif. Un moteur 6 est prévu pour entraîner en rotation le rotor 4.

La chambre de pompage 3 communique avec une enceinte 7, dont la partie inférieure forme un bac à huile de lubrification (non visible). Dans la partie supérieure de l'enceinte 7 est montée un filtre à huile 8 prévu pour extraire l'huile présente dans les gaz pompés avant l'évacuation de ces gaz par exemple dans l'atmosphère, par le refoulement 9 de la pompe à vide 1.

Lorsque le rotor 4 tourne sur son axe, les palettes 5 entraînent les gaz présents dans la chambre de pompage 3 et les expulsent vers l'enceinte 7. Cela génère une aspiration par le sous-ensemble amont 2.

Ici comme dans les revendications annexées, les termes « amont » et « aval », ainsi que les termes analogues, se réfèrent au sens dans lequel s'écoulent les gaz pompés par la pompe à vide 1.

Le sous ensemble-amont 2 comporte un assemblage de plusieurs pièces successives 10a et 10b formant un conduit 10, dont l'extrémité amont est l'aspiration 11 de la pompe à vide 1. L'extrémité aval du conduit 10 communique avec la chambre de pompage 3. Une grille 12 de filtration des gaz aspirés entrant par l'aspiration 11 est montée dans le conduit 10. Une soupape antiretour 13 est montée en aval de la grille 12, dans le conduit 10.

Le sous ensemble-amont 2 est placé en amont de la chambre de pompage 3. Dans un cas particulier des modes de réalisation de l'invention, le sous ensemble-amont 2 est placé directement en amont de la chambre de pompage 3.

La pièce 10a est pourvue de plusieurs éléments de stérilisation dont chacun est une source de lumière ultraviolette 15. Par exemple, chaque source de lumière ultraviolette 15 peut être une LED. Les sources de lumière ultraviolette 15 sont disposées et dirigées de manière à éclairer ensemble la totalité de la paroi intérieure 16 de la pièce 10a, ainsi que la surface amont 17 de la grille 12. Les faisceaux de lumière ultraviolette émis par les sources de lumière ultraviolette 15 sont symbolisés par les flèches F sur la figure 1.

La zone délimitée par la pièce 10a est une zone tampon 18 que la lumière ultraviolette émise par les sources de lumière ultraviolette 15 aseptise dans sa totalité en détruisant les éventuels microorganismes présents et les éventuels virus présents. La zone tampon 18 forme un passage par lequel s'écoule la totalité des gaz pompé par la pompe à vide 1. La zone tampon est ainsi une zone intermédiaire entre une région amont et une région aval le long de la trajectoire des gaz entraînés par le pompage par la pompe à vide 1. La zone tampon 18 se trouve en amont de la chambre de pompage 3, ainsi que de la grille 12, et s'étend jusqu'à l'aspiration 11.

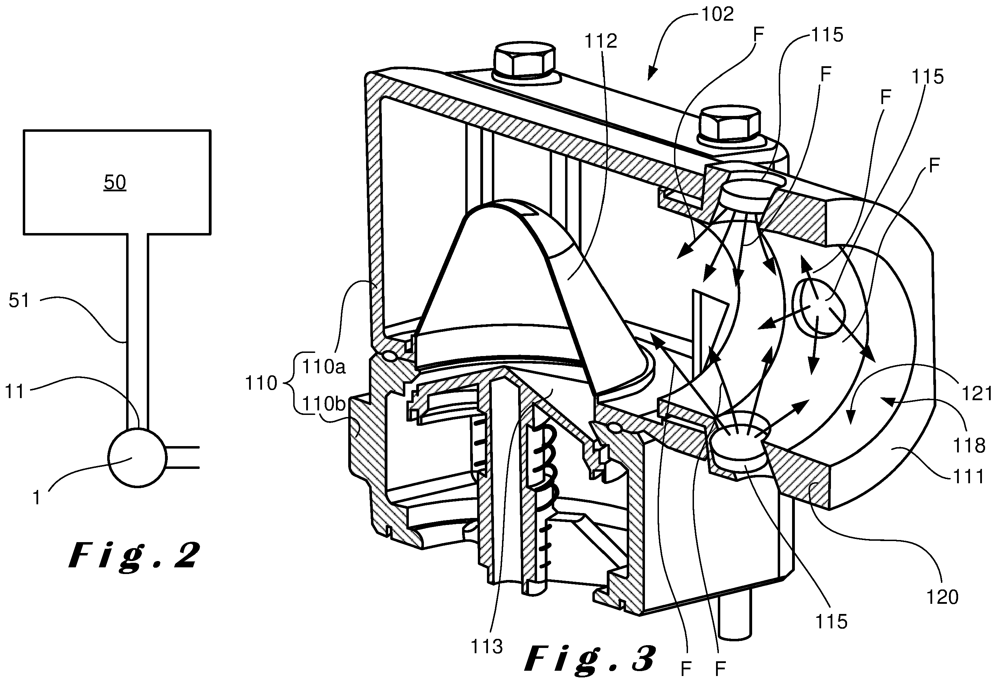

Sur la figure 2, une installation conforme à l'invention est une installation de traitement et/ou d'emballage de produits alimentaires. Elle comprend une chambre à vide 50, la pompe à vide 1, ainsi qu'un conduit de raccordement 51 qui raccorde une ouverture de la chambre à vide 50 à l'aspiration 11 de la pompe à vide 1. Dans la chambre à vide 50, on emballe sous vide des produits alimentaires. Dans la chambre à vide 50, on peut également traiter des produits alimentaires et, éventuellement, les emballer sous vide ensuite.

Une contamination éventuelle par exemple par une bactérie en amont de la zone tampon 18 ne peut pas franchir cette zone tampon 18, vers l'aspiration 11 et vers un conduit de raccordement tel que le conduit de raccordement 51 raccordant la chambre à vide 50 à la pompe à vide 1 dans le cas de l'installation de traitement et/ou d'emballage représentée sur la figure 2. Dans ce cas, la zone tampon 18 empêche ainsi une contamination de la chambre à vide 50 depuis la pompe à vide 1, par un micro-organisme pathogène.

En outre, si des virus par exemple provenant d'aliments dans la chambre à vide 50 sont entraînés par les gaz aspirés vers la pompe à vide 1, ces virus sont détruits dans la zone tampon 18 et ne sont ainsi pas expulsés dans l'atmosphère environnant, par le refoulement 9.

Si de la matière alimentaire en provenance de la chambre à vide 50 se trouve sur la grille 12 après avoir été arrêtée par cette grille 12, elle reçoit un rayonnement ultraviolet depuis une partie au moins des sources de lumière ultraviolette 15. Cette matière alimentaire présente sur la grille 12 est ainsi aseptisée et ne peut pas devenir un foyer de développement de microorganismes et de contamination d'autres régions telles que le conduit de raccordement 51 et la chambre à vide 50, par de tels microorganismes.

Un sous-ensemble amont 102 d'une pompe à vide selon un deuxième mode de réalisation de l'invention est représentée sur la figure 3. Dans ce qui suit, on ne décrit que ce par quoi la pompe à vide selon le deuxième mode de réalisation de l'invention se distingue de la pompe à vide 1. En outre, lorsqu'une partie référencée de la pompe à vide selon le deuxième mode de réalisation de l'invention est identique ou équivalente à une partie référencée de la pompe à vide 1, sa référence est construite en ajoutant 100 à la référence désignant cette partie référencée sur la pompe à vide 1. De cette manière sont construites notamment les références de la grille 112 et de la soupape antiretour 113.

En plus des parties 110a et 110b, le conduit 110 du sous-ensemble amont 102 comporte un conduit rapporté 120, lequel est pourvu des sources de lumière ultraviolette 115. Le conduit rapporté 120 comporte l'aspiration 111 de la pompe à vide selon le deuxième mode de réalisation de l'invention. Dans l'exemple représenté, aucune source de lumière ultraviolette n'équipe la partie 110a. Lorsque tel est le cas, le conduit rapporté 120 peut être monté sur une pompe à vide initialement dépourvue de source de lumière ultraviolette 115 et ainsi munir cette pompe à vide de sources de lumière ultraviolette 115 et d'une zone tampon 118.

La zone tampon 118 comprend le passage délimité par le conduit rapporté 120 et s'étend jusqu'à l'aspiration 111. La paroi intérieure 121 du conduit rapporté 120 est entièrement éclairée par les sources de lumière ultraviolette 115. Toute la zone tampon 118 y compris cette paroi intérieure 121 est ainsi aseptisée par un rayonnement ultraviolet. Une contamination éventuelle par un microorganisme en amont de la zone tampon 118 ne peut pas franchir cette zone tampon 118, vers l'aspiration 111 et vers une chambre à vide lorsqu'une telle chambre à vide est raccordée à cette aspiration 111.

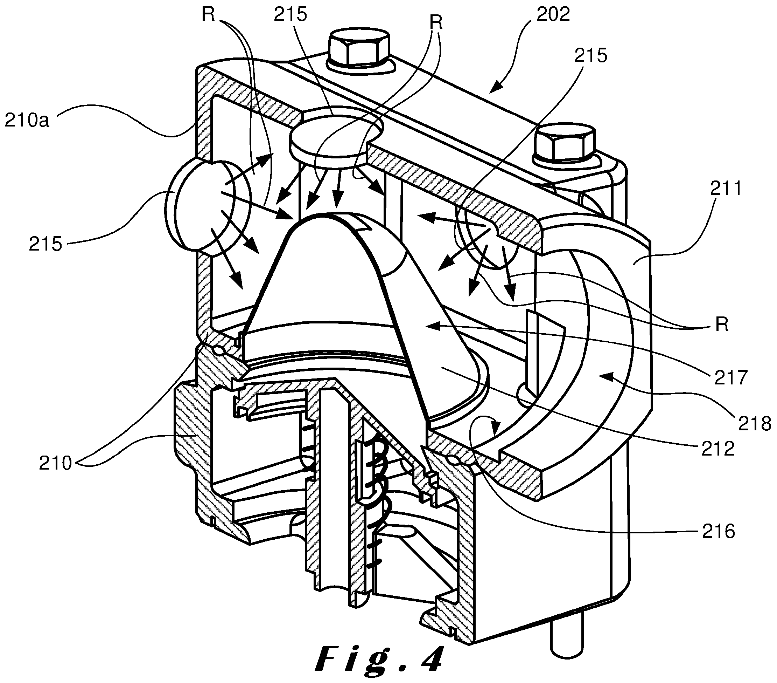

Un sous-ensemble amont 202 d'une pompe à vide selon un troisième mode de réalisation de l'invention est représentée sur la figure 4. Dans ce qui suit, on ne décrit que ce par quoi la pompe à vide selon le troisième mode de réalisation de l'invention se distingue de la pompe à vide 1. En outre, lorsqu'une partie référencée de la pompe à vide selon le troisième mode de réalisation de l'invention est identique ou équivalente à une partie référencée de la pompe à vide 1, sa référence est construite en ajoutant 200 à la référence désignant cette partie référencée sur la pompe à vide 1.

Comme la partie 10a, la partie 210a du conduit 210 est pourvue d'éléments de stérilisation. Au lieu d'être d'une source de lumière ultraviolette 15, chacun de ces éléments de stérilisation est un élément chauffant 215. Les éléments chauffants 215 sont à même de chauffer par rayonnement la paroi intérieure 216 de la partie 210a et la surface amont 217 de la grille 212 afin de les porter à une température à laquelle sont détruits au moins certains micro-organismes. Par exemple, cette température peut être supérieure à environ 50°C. Elle est de préférence supérieure à environ 70°C et, de manière encore plus préférée, supérieure à environ 120°C. Toute la zone tampon 218 y compris la paroi intérieure 216 et la surface amont 217 est ainsi stérilisée jusqu'à l'aspiration 211. Le rayonnement émis par les éléments chauffant 215 est symbolisé par les flèches R sur la figure 4.

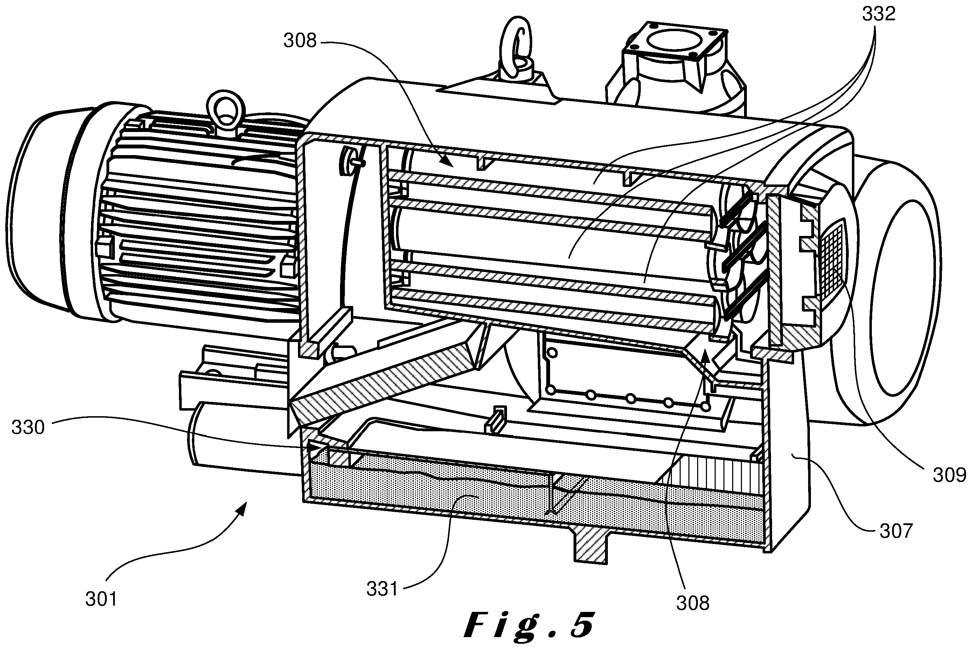

Une pompe à vide 301 selon un quatrième mode de réalisation de l'invention est représentée sur la figure 5. Dans ce qui suit, on ne décrit que ce par quoi la pompe à vide 301 se distingue de la pompe à vide 1. En outre, lorsqu'une partie référencée de la pompe à vide 301 est identique ou équivalente à une partie référencée de la pompe à vide 1, sa référence est construite en ajoutant 300 à la référence désignant cette partie référencée sur la pompe à vide 1. Par exemple, la référence du refoulement 309 de la pompe à vide 301 est construite ainsi.

Comme dans le premier mode de réalisation de l'invention, la partie inférieure de l'enceinte 307 forme un bac 330 qui est un bac à huile de lubrification et qui sert à récupérer et stocker l'huile 331 de lubrification des palettes (non visibles) de la pompe à vide 301 d'une manière connue en soi dans les pompes lubrifiées à palettes. Toujours comme dans le premier mode de réalisation, la partie supérieure de l'enceinte 307 renferme un filtre à huile 308 prévu pour extraire l'huile de lubrification 331 présente dans les gaz après leur entraînement par les palettes (non visibles) dans la chambre de pompage (non visible). L'huile de lubrification 331 extraite par le filtre à huile 308 s'écoule par gravité jusqu'au bac 330.

L'huile de lubrification 331 contient un élément de stérilisation qui est un produit chimique antiseptique à même de supprimer un ou plusieurs micro-organisme et/ou un ou plusieurs virus. Par exemple, ce produit chimique antiseptique peut être du p-Chloro-m-cresol (PCMC) ou tout autre produit chimique pouvant convenir comme biocide tels que o-Phenylphenol (OPP), lodopropynylbutylcarbamate (IPBC), Benzisothiazolinone (BIT) ou Bronopol. En variante, l'huile de lubrification 331 peut être elle-même, de par sa composition, l'élément de stérilisation. L'huile de lubrification 331 stérilise une zone tampon 318 qui comprend la chambre de pompage et la région comprenant les cartouches filtrantes 332 du filtre à huile 308. Des virus éventuellement aspirés par la pompe à vide 301 sont détruits au niveau de la zone tampon 318 et ne sont ainsi pas rejetés dans l'atmosphère environnant par le refoulement 309.

La pompe à vide 301 peut comprendre des sources de lumière ultraviolette comme dans le premier mode de réalisation de l'invention, auquel cas ces sources de lumière ultraviolette peuvent être agencées et orientées comme les sources de lumière ultraviolette 15 le sont dans le premier mode de réalisation ou comme les sources de lumière ultraviolettes 115 le sont dans le deuxième mode de réalisation de l'invention. La pompe à vide 301 peut également comprendre des éléments chauffants pouvant être agencés et orientés comme les éléments chauffants 215 le sont dans le troisième mode de réalisation de l'invention. La pompe à vide peut aussi ne comprendre ni source de lumière ultraviolette, ni élément chauffant.

Dans une pompe à vide non représentée selon un cinquième mode de réalisation de l'invention, des sources de lumière ultraviolette peuvent être prévus dans une enceinte ayant la même fonction que l'enceinte 307 du quatrième mode de réalisation de l'invention. Dans ce cas, ces sources de lumière ultraviolette peuvent être prévue dans la partie inférieure de l'enceinte, de manière à pouvoir éclairer l'huile de lubrification et le bac à huile, ou dans la partie supérieure de l'enceinte, par exemple à la place d'une de plusieurs cartouches du filtre à huile.

L'invention ne se limite pas aux modes de réalisation décrits précédemment. En particulier, bien que la pompe à vide dans ces modes de réalisation soit une pompe lubrifiée à palettes, l'invention ne se limite pas aux pompes lubrifiées à palettes. En effet, tout type de pompe à vide, lubrifiée ou non lubrifiée, peut être conforme à l'invention. En particulier, une pompe à vide conforme à l'invention peut être choisie parmi une pompe à palettes, une pompe à vis, une pompe à engrenages, un éjecteur et une pompe à lobes.

## Revendications

1. Pompe à vide, comportant une aspiration (11 ; 111 ; 211), un refoulement (9 ; 309) et un volume intérieur prévu pour être parcouru par les gaz entraînés de l'aspiration (11 ; 111 ; 211) au refoulement (9 ; 309) par le pompage par la pompe à vide,
**caractérisée en ce qu'**elle comprend au moins un élément de stérilisation (15 ; 115 ; 215 ; 331) d'au moins une portion (18 ; 118 ; 218 ; 318) du volume intérieur ;
et **en ce que** le volume intérieur comporte une zone tampon (18 ; 118 ; 218 ; 318) stérilisable par le ou les éléments de stérilisation (15 ; 115 ; 215 ; 331), cette zone tampon (18 ; 118 ; 218 ; 318) comprenant un passage pour l'ensemble des gaz entraînés par le pompage par la pompe à vide.

2. Pompe à vide selon la revendication 1, **caractérisée en ce que** l'élément de stérilisation (15 ; 115 ; 215 ; 331) est l'un de plusieurs éléments de stérilisation (15 ; 115 ; 215 ; 331) que comporte la pompe à vide.

3. Pompe à vide selon la revendication 1 ou 2, **caractérisée en ce que** la zone tampon (18 ; 118 ; 218) s'étend jusqu'à l'aspiration (11 ; 111 ; 211) de la pompe à vide.

4. Pompe à vide selon la revendication 1 ou 3, **caractérisée en ce qu'**elle comprend au moins une chambre de pompage (3) où s'effectue l'entraînement des gaz par la pompe à vide, au moins une partie du passage de la zone tampon (18 ; 118 ; 218) se trouvant en amont de la chambre de pompage (3).

5. Pompe à vide selon l'une quelconque des revendications 1 à 4, **caractérisée en ce qu'**elle comprend une grille (12 ; 112) de filtration des gaz pompés, cette grille (12 ; 112) se trouvant en amont de la chambre de pompage (3), au moins une partie du passage de la zone tampon (18 ; 118 ; 218) se trouvant en amont de la grille (12 ; 112).

6. Pompe à vide selon la revendication 5, **caractérisée en ce qu'**une surface amont (17 ; 217) parmi deux surfaces principales opposées de la grille (12 ; 112) est stérilisable par le ou les éléments de stérilisation (15 ; 215).

7. Pompe à vide selon l'une quelconque des revendications précédentes, **caractérisée en ce qu'**elle comprend un conduit rapporté (120) formant l'aspiration (111) de la pompe à vide, ce conduit rapporté (120) étant pourvu d'au moins un élément de stérilisation (115).

8. Pompe à vide selon l'une quelconque des revendications précédentes, **caractérisée en ce qu'**au moins l'un des éléments de stérilisation est une source de lumière ultraviolette (15 ; 115).

9. Pompe à vide selon la revendication 8, **caractérisée en ce que** la source de lumière ultraviolette est l'une de plusieurs sources de lumière ultraviolette (15 ; 115) que comporte la pompe à vide et qui sont à même d'éclairer ensemble toute une portion de paroi intérieure (16, 17 ; 121) délimitant et entourant le passage de la zone tampon (18 ; 118).

10. Pompe à vide selon l'une quelconque des revendications précédentes, **caractérisée en ce qu'**au moins l'un des éléments de stérilisation est un élément chauffant (215).

11. Pompe à vide selon l'une quelconque des revendications précédentes, **caractérisée en ce que** la pompe à vide est une pompe lubrifiée comprenant une chambre de pompage où s'effectue l'entraînement des gaz par la pompe à vide, ainsi que de l'huile de lubrification (331) présente notamment dans la chambre de pompage, l'huile de lubrification (331) étant ou comprenant au moins un des éléments de stérilisation.

12. Pompe à vide selon la revendication 11, **caractérisée en ce que** l'élément de stérilisation est ou comprend un produit chimique antiseptique.

13. Installation de traitement et/ou d'emballage de produits alimentaires, comprenant une chambre à vide (50), **caractérisée en ce qu'**elle comprend au moins une pompe à vide (1 ; 301) selon l'une quelconque des revendications précédentes, l'aspiration (11 ; 111 ; 211) de cette pompe à vide (1 ; 301) étant raccordée à la chambre à vide (50).

## Patentansprüche

1. Vakuumpumpe umfassend einen Ansaugbereich (11; 111; 211), einen Auslass (9; 309) und ein Innenvolumen, welches dazu eingerichtet ist, mittels Pumpen mit der Vakuumpumpe, von den, von dem Ansaugbereich (11; 111; 211) zum Auslass (9; 309) geförderten, Gasen, durchströmt zu werden,
**dadurch gekennzeichnet, dass** sie mindestens ein Sterilisationselement (15; 115; 215; 331) für mindestens einen Teil (18; 118; 218; 318) des Innenvolumens umfasst;
und dass der Innenraum einen Pufferbereich (18; 118; 218; 318) umfasst, der durch das Sterilisationselement oder die Sterilisationselemente (15; 115; 215; 331) sterilisierbar ist, welcher Pufferbereich (18; 118; 218; 318) einen Durchgang für die Gesamtheit der durch das Pumpen mit der Vakuumpumpe geförderten Gase umfasst.

2. Vakuumpumpe nach Anspruch 1, **dadurch gekennzeichnet, dass** das Sterilisationselement (15; 115; 215; 331) eines von mehreren Sterilisationselementen ist (15; 115; 215; 331) ist, die die Vakuumpumpe umfasst.

3. Vakuumpumpe nach Anspruch 1 oder 2, **dadurch gekennzeichnet, dass** sich der Pufferbereich (18; 118; 218) bis zum Ansaugbereich (11; 111; 211) der Vakuumpumpe erstreckt.

4. Vakuumpumpe nach Anspruch 1 oder 3, **dadurch gekennzeichnet, dass** sie mindestens eine Pumpenkammer (3) umfasst, in der die Gase durch die Vakuumpumpe gepumpt werden, wobei sich mindestens ein Teil des Durchgangs der Pufferzone (18; 118; 218) stromaufwärts der Pumpenkammer (3) befindet.

5. Vakuumpumpe nach einem der Ansprüche 1 bis 4, **dadurch gekennzeichnet, dass** sie ein Gitter (12; 112) zum Filtern der Gase umfasst, wobei sich das Gitter (12; 112) stromaufwärts der Pumpenkammer (3) befindet und mindestens ein Teil des Durchgangs der Pufferzone (18; 118; 218) sich stromaufwärts des Gitters (12; 112) befindet.

6. Vakuumpumpe nach Anspruch 5, **dadurch gekennzeichnet, dass** eine stromaufwärtige Fläche (17; 217) von zwei gegenüberliegenden Hauptflächen des Gitters (12; 112) durch das Sterilisationselement oder die Sterilisationselemente (15; 215) sterilisierbar.

7. Vakuumpumpe nach einem der vorstehenden Ansprüche, **dadurch gekennzeichnet, dass** sie eine Ansaugleitung (120) umfasst, die den Ansaugbereich (111) der Vakuumpumpe bildet, wobei diese Ansaugleitung (120) mit mindestens einem Sterilisationselement (115) versehen ist.

8. Vakuumpumpe nach einem der vorstehenden Ansprüche, **dadurch gekennzeichnet, dass** mindestens eines der Sterilisationselemente eine Ultraviolettlichtquelle (15; 115) ist.

9. Vakuumpumpe nach Anspruch 8, **dadurch gekennzeichnet, dass** die Ultraviolettlichtquelle eine von mehreren Ultraviolettlichtquellen (15; 115) ist, die in der Lage sind, gemeinsam einen gesamten Abschnitt der Innenwand (16, 17; 121) zu beleuchten, der den Durchgang der Pufferzone (18; 118) begrenzt und umgibt.

10. Vakuumpumpe nach einem der vorstehenden Ansprüche, **dadurch gekennzeichnet, dass** mindestens eines der Sterilisationselemente ein Heizelement ist (215).

11. Vakuumpumpe nach einem der vorstehenden Ansprüche, **dadurch gekennzeichnet, dass** die Vakuumpumpe eine geschmierte Pumpe ist, die eine Pumpenkammer umfasst, in der die Gase durch die Vakuumpumpe gefördert werden, sowie Schmieröl (331), das insbesondere in der Kammer vorhanden ist, wobei das Schmieröl (331) mindestens eines der Sterilisationselemente ist oder enthält.

12. Vakuumpumpe nach Anspruch 11, **dadurch gekennzeichnet, dass** das Sterilisationselement eine antiseptische Chemikalie ist oder enthält.

13. Anlage zur Behandlung und/oder Verpackung von Lebensmitteln, mit einer Vakuumkammer (50), **dadurch gekennzeichnet, dass** sie mindestens eine Vakuumpumpe (1; 301) gemäß einem der vorstehenden Ansprüche umfasst, wobei der Ansaugbereich (11; 111; 211) dieser Vakuumpumpe (1; 301) mit der Vakuumkammer (50) verbunden ist.

## Claims

1. Vacuum pump, comprising a suction (11; 111; 211), a discharge (9; 309) and an internal volume through which the gases conveyed from the suction (11; 111; 211) to the discharge (9; 309) by the pumping of the vacuum pump are to flow,
**characterized in that** it comprises at least one sterilization element (15; 115; 215; 331) of at least one portion (18; 118; 218; 318) of the interior volume;
and **in that** the interior volume comprises a buffer zone (18; 118; 218; 318) that is able to be sterilized by the sterilization element(s) (15; 115; 215; 331), this buffer zone (18; 118; 218; 318) comprising a passage for all the gases conveyed by the pumping by the vacuum pump.

2. Vacuum pump according to claim 1, **characterized in that** the sterilization element (15; 115; 215; 331) is one of a plurality of sterilization elements (15; 115; 215; 331) that the vacuum pump comprises.

3. Vacuum pump according to claim 1 or 2, **characterized in that** the buffer zone (18; 118; 218) extends to the suction (11; 111; 211) of the vacuum pump.

4. Vacuum pump according to claim 1 or 3, **characterized in that** it comprises at least one pumping chamber (3) where the conveying of the gases by the vacuum pump takes place, at least a part of the passage of the buffer zone (18; 118; 218) being located upstream of the pumping chamber (3).

5. Vacuum pump according to any one of the claims 1 to 4, **characterized in that** it comprises a grid (12; 112) for filtering the pumped gases, this grid (12; 112) being located upstream of the pumping chamber (3), at least part of the passage of the buffer zone (18; 118; 218) being located upstream of the grid (12; 112).

6. Vacuum pump according to claim 5, **characterized in that** one upstream surface (17; 217) of two opposing major surfaces of the grid (12; 112) is sterilizable by the sterilizing element(s) (15; 215).

7. Vacuum pump according to any one of the preceding claims, **characterized in that** it comprises an insert duct (120) forming the suction (111) of the vacuum pump, this insert duct (120) being provided with at least one sterilization element (115).

8. Vacuum pump according to any one of the preceding claims, **characterized in that** at least one of the sterilization elements is an ultraviolet light source (15; 115).

9. Vacuum pump according to claim 8, **characterized in that** the ultraviolet light source is one of several ultraviolet light sources (15; 115) included in the vacuum pump and which are able to illuminate together a whole portion of the inner wall (16, 17; 121) delimiting and surrounding the passage of the buffer zone (18; 118).

10. Vacuum pump according to any one of the preceding claims, **characterized in that** at least one of the sterilization elements is a heating element (215).

11. Vacuum pump according to any one of the preceding claims, **characterized in that** the vacuum pump is a lubricated pump comprising a pumping chamber where the conveying of gases by the vacuum pump takes place, as well as lubricating oil (331) present in particular in the pumping chamber, the lubricating oil (331) being or comprising at least one of the sterilization elements.

12. Vacuum pump according to claim 11, **characterized in that** the sterilization element is or comprises an antiseptic chemical substance.

13. Installation for processing and/or packaging food products, comprising a vacuum chamber (50), **characterized in that** it comprises at least one vacuum pump (1; 301) according to any of the preceding claims, the suction (11; 111; 211) of this vacuum pump (1; 301) being connected to the vacuum chamber (50).
